# EUROPEAN PATENT APPLICATION

(11) **EP 3 903 869 A1**
(43) Date of publication of application: **03.11.2021**
(21) Application number: 19905419.8
(22) Date of filing: 25.12.2019
(51) Int. Cl.: A61M 21/02, G01S 13/58, A61B 5/11, A61B 5/16

(54) **INFORMATION PROCESSING DEVICE, INFORMATION PROCESSING METHOD, INFORMATION PROCESSING PROGRAM, AND INFORMATION PROCESSING SYSTEM**

(30) Priority: 28.12.2018 JP 2018248119
(71) Applicant: Minami, Noriyuki, Kyoto-shi, Kyoto 605-0983 (JP)
(72) Inventor: HATTORI, Yurie, Kyoto-shi, Kyoto 605-0983 (JP); KAWAMURA, Kazuhiro, Kyoto-shi, Kyoto 605-0983 (JP); SAITO, Koji, Kyoto-shi, Kyoto 605-0983 (JP)
(74) Representative: Bardehle Pagenberg Partnerschaft mbB Patentanwälte Rechtsanwälte
(86) International application number: PCT/IB2019/061351
(87) International publication number: WO 2020/136590

(57) **Abstract**

An information processing apparatus includes a sensing unit that senses body motion of a user present within a prescribed area, a determination unit that determines whether or not the user is in a ready-to-sleep state based on a result of sensing by the sensing unit, and a processing performing unit that performs first processing when a result of determination by the determination unit indicates that the user has entered the ready-to-sleep state.

## Description

### TITLE OF INVENTION

Information Processing Apparatus, Information Processing Method, Information Processing Program, and Information Processing System

### TECHNICAL FIELD

The present disclosure relates to an information processing apparatus that manages sleep of a user.

### BACKGROUND ART

An apparatus that determines a sleep state of a user based on information on motion of a body of a user has conventionally been proposed (see PTL 1).

### CITATION LIST

### PATENT LITERATURE

PTL 1: Japanese Patent Laying-Open No. 2014-14708

### SUMMARY OF INVENTION

### TECHNICAL PROBLEM

The conventional apparatus simply determines the sleep state of the user and starts performing prescribed processing based on a result of determination, and it has not managed falling asleep and sleep of the user as a whole.

The present disclosure was made to solve a problem as above, and an object of the present disclosure is to provide an information processing apparatus, an information processing method, an information processing program, and an information processing system capable of managing falling asleep and sleep of a user as a whole.

### SOLUTION TO PROBLEM

An information processing apparatus according to one aspect includes a sensing unit that senses body motion of a user present within a prescribed area, a determination unit that determines whether or not the user is in a ready-to-sleep state based on a result of sensing by the sensing unit, and a processing performing unit that performs first processing when a result of determination by the determination unit indicates that the user has entered the ready-to-sleep state.

When the result of determination by the determination unit indicates that the user has entered the ready-to-sleep state, the processing performing unit performs first processing. Therefore, the ready-to-sleep state of the user can be sensed and the first processing can be performed to induce the user to sleep. Therefore, falling asleep and sleep of the user as a whole can be managed.

Preferably, the determination unit may determine that the user has entered the ready-to-sleep state based on at least one of sensing of the user within the prescribed area and the user being in a lying-to-sleep state based on the result of sensing by the sensing unit.

Since the determination unit makes determination as the ready-to-sleep state in response to sensing of the user within the prescribed area or the user being in the lying-to-sleep state, the state of the user for readiness to sleep of the user is reliably sensed. Therefore, falling asleep and sleep of the user as a whole can be managed.

Preferably, the information processing apparatus further includes an ambient environment obtaining unit that obtains a state of an environment around the information processing apparatus. When the result of determination by the determination unit indicates that the user has entered the ready-to-sleep state and when the state of the environment obtained by the ambient environment obtaining unit satisfies an ambient environment condition, the processing performing unit performs the first processing.

When the state of the environment obtained by the ambient environment obtaining unit satisfies the ambient environment condition, the first processing is performed. By reliably sensing the state of readiness for sleep of the user based on combination with the ambient environment condition, falling asleep and sleep of the user as a whole can be managed.

Preferably, the ambient environment obtaining unit may obtain as the state of the environment, information on ambient brightness.

By using information on ambient brightness, the state of readiness for sleep of the user can reliably be estimated.

Preferably, the information on ambient brightness includes an illuminance value.

By using the illuminance value, the state of readiness for sleep of the user can readily be estimated.

Preferably, the information processing apparatus further includes a communication unit provided to communicate with an external apparatus. The processing performing unit may provide audio output of information on a next day or a next morning from the external apparatus through the communication unit, as the first processing.

In the first processing, information on the next day or the next morning is obtained and audio output of the obtained information is provided. Therefore, information highly convenient for the user is provided, which can contribute to convenience of the user.

Preferably, the information processing apparatus further includes a notification unit that performs a notification operation when time set in advance comes. The information on the next day or the next morning includes time at which the notification operation is performed.

In the first processing, time of the notification operation on the next day or in the next morning is provided. Therefore, information highly convenient for the user is provided, which can contribute to convenience of the user.

Preferably, the information processing apparatus further includes a voice recognition unit that accepts audio input and carries out voice recognition. The processing performing unit changes time at which the notification operation is performed, in accordance with a result of the voice recognition by the voice recognition unit.

Therefore, time to perform the notification operation can be changed in response to an audio instruction from the user, which can contribute to convenience of the user.

Preferably, the information on the next day or the next morning includes information on weather on the next day or in the next morning. In the first processing, information on the weather on the next day or in the next morning is provided. Therefore, information highly convenient for the user is provided, which can contribute to convenience of the user.

Preferably, the sensing unit includes a Doppler sensor. The information processing apparatus further includes a body motion sensing unit that senses body motion of the user based on an output from the Doppler sensor.

The sensing unit senses body motion of the user, determination as to the ready-to-sleep state is made based on a result of sensing, and the first processing is performed based on a result of the determination. Therefore, the first processing can readily be performed without giving an instruction to the apparatus.

Preferably, the information processing apparatus further includes a sleep state measurement unit that measures a sleep state of the user following the first processing, based on the output from the Doppler sensor.

Therefore, since the sleep state measurement unit measures the sleep state of the user, sleep of the user can be managed.

Preferably, when the user is determined as having entered a rest state based on measurement of the rest state of the user, the processing performing unit performs second processing for adjusting contents of the first processing.

Therefore, the second processing is performed based on measurement of the rest state of the user, and hence falling asleep and sleep of the user as a whole can be managed.

Preferably, the processing performing unit performs third processing for inducing the user to the sleep state when the user is not in the sleep state, and the processing performing unit stops the third processing when the user enters the sleep state.

Therefore, by stopping the third processing, falling asleep and sleep of the user as a whole can be managed.

Preferably, the third processing may include music play processing.

Therefore, the music play processing is performed to induce the user to sleep, and falling asleep and sleep of the user as a whole can be managed.

Preferably, the third processing may include voice guidance processing for indicating a breathing method or prescribed exercise.

Therefore, the voice guidance processing to indicate the breathing method or prescribed exercise is performed to induce the user to sleep, and falling asleep and sleep as a whole can be managed.

Preferably, the sensing unit may sense body motion of the user present within the prescribed area over the whole period of twenty-four hours.

Since the sensing unit senses body motion of the user over the whole period of twenty-four hours, falling asleep and sleep of the user as a whole can be managed.

An information processing method according to one aspect includes sensing body motion of a user present within a prescribed area, determining whether or not the user is in a ready-to-sleep state based on a result of sensing, and performing first processing when the user is determined as having entered the ready-to-sleep state.

When the user is determined as having entered the ready-to-sleep state in the determining step, first processing is performed. Therefore, the ready-to-sleep state of the user can be sensed, and the first processing can be performed to induce the user to sleep. Therefore, falling asleep and sleep of the user as a whole can be managed.

An information processing program according to one aspect executed by a computer including a sensor that senses a signal in accordance with motion of a user causes the computer to perform sensing body motion of the user present within a prescribed area based on the signal from the sensor, determining whether or not the user is in a ready-to-sleep state based on a result of sensing, and performing first processing when the user is determined as having entered the ready-to-sleep state.

When the user is determined as having entered the ready-to-sleep state in the determining step, first processing is performed. Therefore, the ready-to-sleep state of the user can be sensed and the first processing can be performed to induce the user to sleep. Therefore, falling asleep and sleep of the user as a whole can be managed.

An information processing system according to one aspect includes a sensing unit that senses body motion of a user present within a prescribed area, a determination unit that determines whether or not the user is in a ready-to-sleep state based on a result of sensing by the sensing unit, and a processing performing unit that performs first processing when a result of determination by the determination unit indicates that the user has entered the ready-to-sleep state.

When the result of determination by the determination unit indicates that the user has entered the ready-to-sleep state, the processing performing unit performs first processing. Therefore, the ready-to-sleep state of the user can be sensed and the first processing can be performed to induce the user to sleep. Therefore, falling asleep and sleep of the user as a whole can be managed.

### ADVANTAGEOUS EFFECTS OF INVENTION

According to the above, the information processing apparatus, the information processing method, the information processing program, and the information processing system in the present disclosure can manage falling asleep and sleep of the user as a whole.

### BRIEF DESCRIPTION OF DRAWINGS

Fig. 1 is a schematic block diagram of a configuration of a sleep management system 1 based on an embodiment.
Fig. 2 is a schematic block diagram of a configuration of a sleep alarm apparatus 2 based on the embodiment.
Fig. 3 is a schematic block diagram of a configuration of a server 6 based on the embodiment.
Fig. 4 is a schematic block diagram of a configuration of a terminal 8 based on the embodiment.
Fig. 5 is a conceptual diagram illustrating a form of use of sleep alarm apparatus 2 based on the embodiment.
Fig. 6 is a diagram illustrating an alarm setting screen set in terminal 8 based on the embodiment.
Fig. 7 is a diagram illustrating processing for starting and quitting a sleep induction function based on the embodiment.
Fig. 8 is a diagram illustrating a functional block diagram of sleep alarm apparatus 2 based on the embodiment.
Fig. 9 is a diagram illustrating a flow of processing for the sleep induction function based on the embodiment.
Fig. 10 is a diagram illustrating a flow of sleep induction function start processing based on the embodiment.
Fig. 11 is a diagram illustrating a flow of other sleep induction function start processing based on the embodiment.
Fig. 12 is a diagram illustrating a flow of processing for performing the sleep induction function based on the embodiment.
Fig. 13 is a diagram illustrating a flow of music play processing based on the embodiment.
Fig. 14 is a diagram illustrating overview of relaxing exercise guidance play processing based on the embodiment.

### DESCRIPTION OF EMBODIMENTS

This embodiment will be described in detail with reference to the drawings. The same or corresponding elements in the drawings have the same reference characters allotted and description thereof will not be repeated.

An information processing apparatus in the present embodiment will be described as a sleep alarm apparatus by way of example. A portable (also referred to as mobile) apparatus or a stationary apparatus may be applicable.

### <Configuration of Sleep Management System>

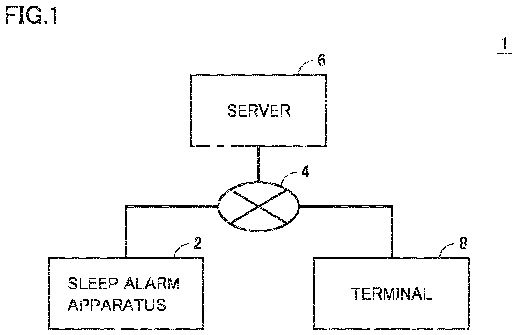

Fig. 1 is a schematic block diagram of a configuration of a sleep management system 1 based on an embodiment.

As shown in Fig. 1, sleep management system 1 includes a sleep alarm apparatus 2, a server 6, and a terminal 8 that are connected to one another over a network 4.

Information can be transmitted and received among sleep alarm apparatus 2, server 6, and terminal 8 over network 4. Network 4 may adopt any of wireless communication and wired communication.

Terminal 8 may be a portable (also referred to as mobile) apparatus such as a portable telephone or a smartphone or a stationary apparatus such as a personal computer.

Sleep alarm apparatus 2 is an apparatus that manages falling asleep and sleep of a user as a whole.

Sleep alarm apparatus 2 performs an alarm function for waking a user up and performs a sensor function to wirelessly sense a signal in accordance with motion of the user. Sleep alarm apparatus 2 performs a sleep induction function for inducing sleep in order to enjoy a comfortable sleep.

Sleep alarm apparatus 2 may also perform a sleep analysis function to analyze sleep data.

Sleep alarm apparatus 2 determines whether or not the user is in a ready-to-sleep state based on a result of sensing by a sensing unit, and when the user is determined as having entered the ready-to-sleep state, it performs prescribed processing.

Terminal 8 can set the alarm function and the sleep induction function of sleep alarm apparatus 2 and can obtain information on a sleep state of the user from sleep alarm apparatus 2 or server 6 and show the information.

Sleep data obtained in sleep alarm apparatus 2 is stored in server 6.

### <Configuration of Sleep Alarm Apparatus 2>

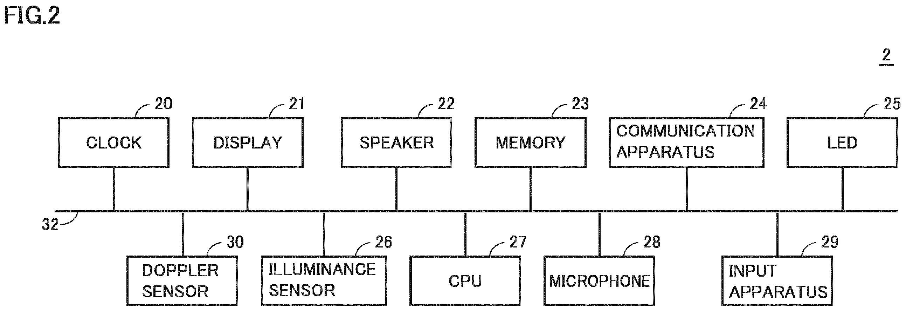

Fig. 2 is a schematic block diagram of a configuration of sleep alarm apparatus 2 based on the embodiment.

As shown in Fig. 2, sleep alarm apparatus 2 includes a clock 20, a display 21, a speaker 22, a memory 23, a communication apparatus 24, an LED 25, an illuminance sensor 26, a CPU 27, a microphone 28, an input apparatus 29, a Doppler sensor 30, and an internal bus 32. Components are connected through internal bus 32.

CPU 27 is an information processing unit that performs various types of information processing performed in sleep alarm apparatus 2. CPU 27 performs various types of information processing by using memory 23. Various programs to be executed in sleep alarm apparatus 2 and data relating to sleep measured in real time when the user sleeps are stored in memory 23.

Though memory 23 is described as a storage contained in sleep alarm apparatus 2, for example, a storage medium attachable to and removable from sleep alarm apparatus 2 such as an optical disc or a cartridge may be applicable or both of the storage and the storage medium as such may be applicable.

CPU 27 implements various functional blocks based on a program stored in memory 23.

Clock 20 performs a function to count time.

Display 21 shows information such as time.

Speaker 22 provides alarm sound as notification sound.

Communication apparatus 24 is an interface for communication with an external apparatus (server 6 and terminal 8) over network 4.

LED 25 is turned on in response to an instruction and lights up an area around sleep alarm apparatus 2.

Microphone 28 accepts external audio input.

Input apparatus 29 includes various operation buttons.

Doppler sensor 30 emits radio waves (microwaves) to an object and wirelessly senses a signal (reflected waves) in accordance with motion of the object (user).

Illuminance sensor 26 detects an illuminance value of a room as information on an ambient environment.

In sleep alarm apparatus 2, the sleep state of the user can be determined as any of four types, for example, of "deep sleep," "light sleep," "REM sleep," and "wake" based on information obtained by detection of reflected waves converted from microwaves emitted from Doppler sensor 30 due to slight motion caused by breathing by the user. A known technique as described, for example, in PTL 1 described previously can be used as a specific method of determination processing. In determining the sleep state, information on slight motion caused by heartbeats may also be taken into account.

Probability as to whether or not a living body is present within an area of monitoring by Doppler sensor 30 can be calculated based on detection of motion caused by breathing described above. For example, whether or not a user is within a monitoring region on the bed can thus be determined.

Sleep alarm apparatus 2 can also detect relatively large body motion such as turn-over or an operation to wave a hand based on a known technique using Doppler sensor 30,. Distinction from slight motion due to breath or heartbeat described above can be made, for example, based on an amount of change between emitted waves and reflected waves or periodicity.

Relatively large motion of the user such as turn-over or an operation to wave a hand may be called "body motion," and such motion, together with slight motion such as breath or heartbeat, may be called "motion."

### <Configuration of Server 6>

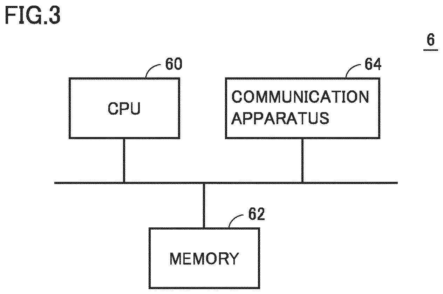

Fig. 3 is a schematic block diagram of a configuration of server 6 based on the embodiment.

As shown in Fig. 3, server 6 includes a CPU 60, a memory 62, a communication apparatus 64, and an internal bus 66. Components are connected through internal bus 66.

CPU 60 is an information processing unit that performs various types of information processing performed in server 6. CPU 60 performs various types of information processing by using memory 62.

Though memory 62 is described as a storage contained in server 6, for example, a storage medium attachable to and removable from server 6 such as an optical disc or a cartridge may be applicable or both of the storage and the storage medium as such may be applicable.

Communication apparatus 64 is an interface for communication with an external apparatus (sleep alarm apparatus 2 and terminal 8) over network 4.

### <Configuration of Terminal 8>

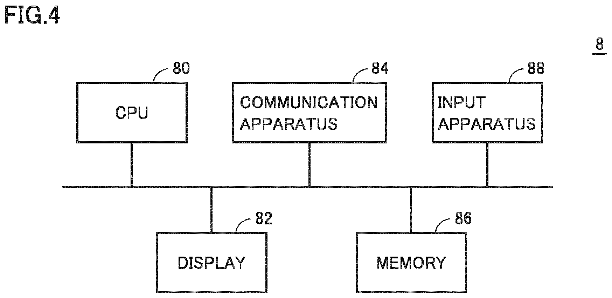

Fig. 4 is a schematic block diagram of a configuration of terminal 8 based on the embodiment.

As shown in Fig. 4, terminal 8 includes a CPU 80, a display 82, a communication apparatus 84, a memory 86, an input apparatus 88, and an internal bus 89. Components are connected through internal bus 89.

CPU 80 is an information processing unit that performs various types of information processing performed in terminal 8. CPU 80 performs various types of information processing by using memory 86.

Input apparatus 88 includes a touch panel.

Communication apparatus 84 is an interface for communication with an external apparatus (sleep alarm apparatus 2 and server 6) over network 4.

Though memory 86 is described as a storage contained in terminal 8, for example, a storage medium attachable to and removable from terminal 8 such as an optical disc or a cartridge may be applicable or both of the storage and the storage medium as such may be applicable.

### <Form of Use of Sleep Alarm Apparatus 2>

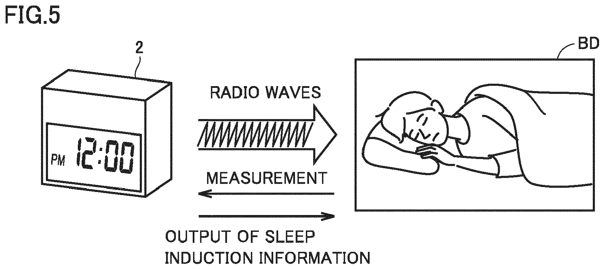

Fig. 5 is a conceptual diagram illustrating a form of use of sleep alarm apparatus 2 based on the embodiment.

As shown in Fig. 5, sleep alarm apparatus 2 is arranged adjacently to a bed BD or the like of a user.

Sleep alarm apparatus 2 measures reflected waves of radio waves emitted from Doppler sensor 30 to a user. The state of the user is monitored based on the reflected waves. A region of monitoring by sleep alarm apparatus 2 corresponds to a prescribed region (prescribed area) where the user sleeps on bed BD.

Sleep alarm apparatus 2 performs the sleep induction function together with the alarm function. In the present example, when sleep alarm apparatus 2 determines the user as being in the ready-to-sleep state, it performs prescribed processing. For example, the sleep alarm apparatus provides sleep induction information. Display 21 shows "PM 12:00" (AM 0:00) as the current time counted by clock 20, by way of example.

### <Alarm Setting Screen>

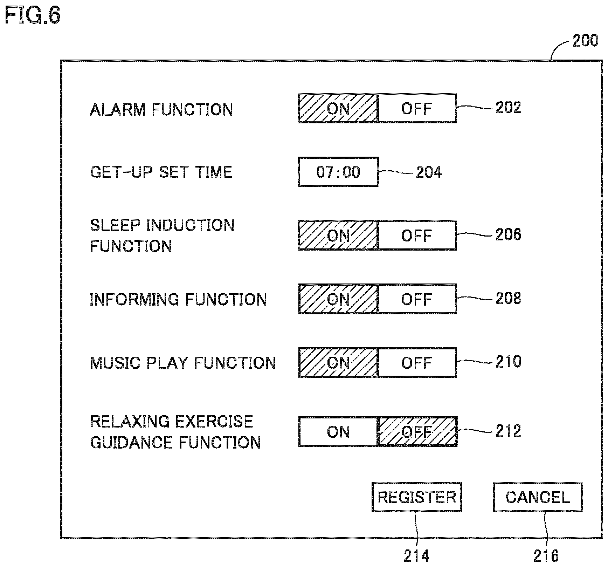

Fig. 6 is a diagram illustrating an alarm setting screen set in terminal 8 based on the embodiment.

Fig. 6 shows an alarm setting screen 200 shown on display 82 of terminal 8. Display 82 of terminal 8 is provided with a touch panel as input apparatus 88, and processing for setting the alarm function of sleep alarm apparatus 2 can be performed through the touch panel. Without being limited to the touch panel, setting processing can also be performed by using a keyboard or the like.

Alarm setting screen 200 is provided with an alarm function on-and-off switch button 202, a get-up set time input field 204, a sleep induction function on-and-off switch button 206, an informing function on-and-off switch button 208, a music play function on-and-off switch button 210, a relaxing exercise guidance function on-and-off switch button 212, a register button 214, and a cancel button 216.

Alarm function on-and-off switch button 202 is a button for switching on or off the alarm function. When the alarm function is on, the notification operation is performed. When the alarm function is off, the prescribed notification operation is not performed. In the present example, an example in which the alarm function is set to "on" is shown. When the alarm function is set to "off", each switch button or selection button or the like is set to an inactive state.

Get-up set time input field 204 is a field where the user enters get-up set time. In the present example, an example in which "07:00" is set in get-up set time input field 204 is shown.

Sleep induction function on-and-off switch button 206 is a button for setting on or off of the function for induction to the sleep state. When the sleep induction function is on, prescribed processing for induction to the sleep state is performed. When the sleep induction function is off, the prescribed processing is not performed. In the present example, an example in which the sleep induction function has been set to "on" is shown.

Informing function on-and-off switch button 208 is a button for switching on or off a function to give information required by the user. When the informing function is on, information giving processing is performed. When the informing function is off, the information giving processing is not performed. In the present example, an example in which the informing function has been set to "on" is shown. Specifically, information required by the user includes information of alarm setting time set by the user and information on weather on the next day or in the next morning.

Music play function on-and-off switch button 210 is a button for setting on or off of the music play function. When the music play function is on, music play processing is performed. When the music play function is off, the music play processing is not performed. In the present example, an example in which the music play function has been set to "on" is shown.

Relaxing exercise guidance function on-and-off switch button 212 is a button for setting on or off of a function to guide relaxing exercise. When the relaxing exercise guidance function is on, exercise guidance processing for guiding relaxing exercise is performed. When the relaxing exercise guidance function is off, the exercise guidance processing is not performed. In the present example, an example in which the relaxing exercise guidance function has been set to "off is shown. The music play function and the relaxing exercise guidance function are not activated in parallel. Therefore, when the music play function is "on", the relaxing exercise guidance function is set to "off".

Register button 214 is a button for performing registration processing for registering in sleep alarm apparatus 2, setting information set in alarm setting screen 200.

Cancel button 216 is a button for canceling registration processing for registering in sleep alarm apparatus 2, setting information set in alarm setting screen 200.

When register button 214 in alarm setting screen 200 shown on display 82 is selected, terminal 8 transmits various types of setting information set in alarm setting screen 100 (which is also simply referred to as "setting information" below) to sleep alarm apparatus 2 over network 4.

Sleep alarm apparatus 2 receives setting information transmitted from terminal 8 and has the setting information stored in memory 23. Sleep alarm apparatus 2 performs the alarm function or the like based on the setting information stored in memory 23.

When cancel button 216 in alarm setting screen 200 shown on display 82 is selected, terminal 8 quits showing alarm setting screen 200 and quits processing for registering the setting information.

Though an example in which processing for setting the alarm function is performed in terminal 8 is described in the present example, without being limited as such, similar setting processing can also be performed by using input apparatus 29 of sleep alarm apparatus 2.

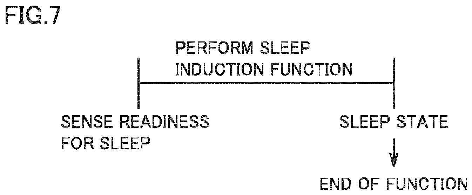

Fig. 7 is a diagram illustrating processing for starting and quitting the sleep induction function based on the embodiment.

As shown in Fig. 7, sleep alarm apparatus 2 senses body motion of the user with Doppler sensor 30. Sleep alarm apparatus 2 makes determination as to the ready-to-sleep state based on a result of sensing by Doppler sensor 30, and when it makes determination as the ready-to-sleep state, it starts the sleep induction function. Sleep alarm apparatus 2 makes determination as to the sleep state based on the result of sensing by Doppler sensor 30, and when it makes determination as the sleep state, it turns off (quits) the sleep induction function.

By way of example of a condition for making determination as the ready-to-sleep state, in the present example, the user is determined as having entered the ready-to-sleep state based on at least one of sensing of the user being within a prescribed area (for example, the bed) and the user having entered the lying-to-sleep state, and then the sleep induction function is started.

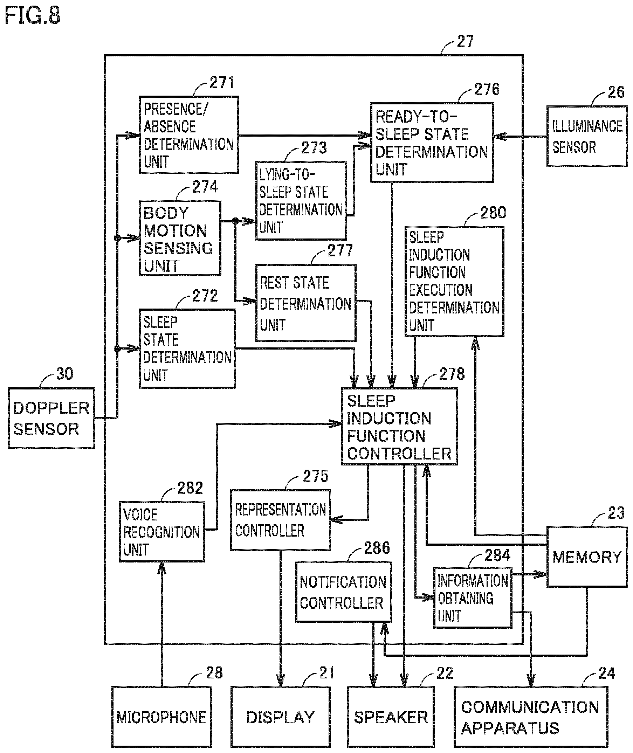

Fig. 8 is a diagram illustrating a functional block diagram of sleep alarm apparatus 2 based on the embodiment.

Referring to Fig. 8, CPU 27 of sleep alarm apparatus 2 implements various functional blocks.

Specifically, CPU 27 implements a plurality of functional blocks based on a program stored in memory 23. In the present example, CPU 27 includes a presence/absence determination unit 271, a sleep state determination unit 272, a lying-to-sleep state determination unit 273, a body motion sensing unit 274, a representation controller 275, a ready-to-sleep state determination unit 276, a rest state determination unit 277, a sleep induction function controller 278, a sleep induction function execution determination unit 280, a voice recognition unit 282, an information obtaining unit 284, and a notification controller 286.

Sleep induction function execution determination unit 280 determines whether or not the sleep induction function of sleep alarm apparatus 2 can be performed. When sleep induction function execution determination unit 280 determines to perform the sleep induction function of sleep alarm apparatus 2, it instructs sleep induction function controller 278 to perform an operation to start the sleep induction function.

Presence/absence determination unit 271 determines whether a user is present or absent (not present) in a prescribed region (prescribed area) corresponding to a monitoring region based on a detection signal from Doppler sensor 30. In the present example, whether the user is present or absent (not present) on the bed is determined.

Sleep state determination unit 272 determines in real time, the sleep state of the user based on a detection signal from Doppler sensor 30. Specifically, sleep state determination unit 272 determines the sleep state of the user as a deep sleep state, a light sleep state, a REM sleep state, or a wake state, based on a detection signal from Doppler sensor 30.

Lying-to-sleep state determination unit 273 makes determination as to the lying-to-sleep state based on an amount of body motion calculated by body motion sensing unit 274. Specifically, when the amount of body motion for a prescribed period is equal to or smaller than a prescribed amount while presence of the user is detected by the Doppler sensor, determination as the lying-to-sleep state is made. A method of making determination as the lying-to-sleep state is not limited thereto, and another approach can naturally be employed. The lying-to-sleep state includes a state that the user is in bed while he/she is awake.

Body motion sensing unit 274 calculates an amount of body motion of the user based on a detection signal from Doppler sensor 30.

Notification controller 286 controls issuance of alarm (the notification operation) through speaker 22. Specifically, by way of example, notification controller 286 obtains get-up set time in the setting information stored in memory 23, and when the notification condition is satisfied, it controls speaker 22 to give alarm (perform the notification operation) at the get-up set time.

Representation controller 275 controls representation on display 21.

Ready-to-sleep state determination unit 276 makes determination as to the ready-to-sleep state based on the result of determination by presence/absence determination unit 271 and the result of determination by lying-to-sleep state determination unit 273. Ready-to-sleep state determination unit 276 provides the result of determination to sleep induction function controller 278.

Rest state determination unit 277 makes determination as to the rest state based on the amount of body motion calculated by body motion sensing unit 274. Specifically, determination as the rest state is made when body motion has not been detected for a certain period of time while the Doppler sensor detects presence of the user. The method of making determination as the rest state is not limited as such, and another approach can also naturally be employed.

Sleep induction function controller 278 performs prescribed processing for inducing the user to sleep in accordance with a notification about the ready-to-sleep state from ready-to-sleep state determination unit 276.

Voice recognition unit 282 identifies audio input from the user provided through microphone 28 and provides the audio input to sleep induction function controller 278.

Information obtaining unit 284 obtains information from an external apparatus through communication apparatus 24 in accordance with an instruction from sleep induction function controller 278. Information obtaining unit 284 has the obtained information stored in memory 23.

### <Sleep Induction Function>

The sleep induction function refers to a function to induce the user to the sleep state. For example, it refers to a function to induce the user to the sleep state by playing music to relax the user or by presenting a breathing method or relaxing exercise. The sleep induction function is processing performed in parallel to the processing for the alarm function.

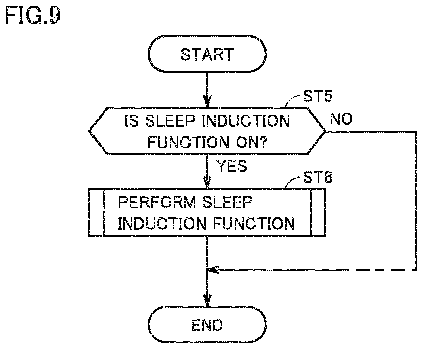

Fig. 9 is a diagram illustrating a flow of processing for the sleep induction function based on the embodiment.

As shown in Fig. 9, CPU 27 determines whether or not the sleep induction function is ON (step ST5). Specifically, sleep induction function execution determination unit 280 determines whether or not the setting information includes information that the sleep induction function is ON.

When CPU 27 determines in ST5 that the sleep induction function is not ON (NO in step ST5), the process ends (end). In this case, the sleep induction function is not performed.

When CPU 27 determines in step ST5 that the sleep induction function is ON (YES in step ST5), it performs sleep induction function start processing (step ST6). When sleep induction function execution determination unit 280 determines that the setting information includes the information that the sleep induction function is ON, it instructs sleep induction function controller 278 to perform the sleep induction function start processing. Details of the sleep induction function start processing will be described later.

Then, the process ends (end).

When the user desires to turn off the sleep induction function (the sleep induction function being OFF) through the processing, the sleep induction function is not performed. Therefore, the processing being performed uselessly can be suppressed and user's intention can be reflected.

### <Sleep Induction Function Start Processing>

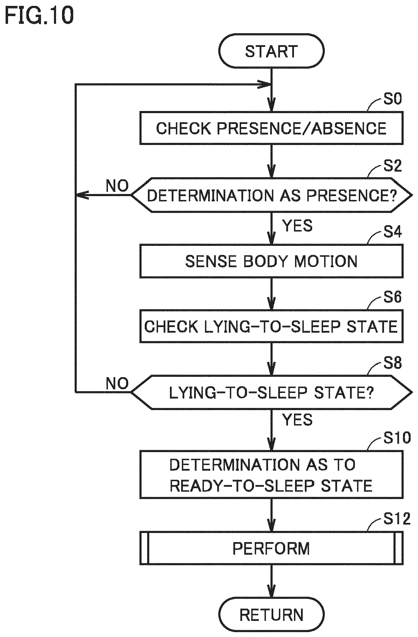

Fig. 10 is a diagram illustrating a flow of the sleep induction function start processing based on the embodiment.

As shown in Fig. 10, CPU 27 checks presence/absence (step S0). Specifically, presence/absence determination unit 271 determines whether or not the user is present within a prescribed region based on a signal from Doppler sensor 30 and provides a result of determination to ready-to-sleep state determination unit 276.

Then, CPU 27 makes determination as to presence (step S2). Ready-to-sleep state determination unit 276 determines whether or not it has received a notification about the result of determination as presence from presence/absence determination unit 271.

Then, when CPU 27 makes determination as presence in step S2 (YES in step S2), it senses body motion (step S4). Body motion sensing unit 274 senses body motion of the user based on a signal from Doppler sensor 30. Body motion sensing unit 274 provides a result of sensing to lying-to-sleep state determination unit 273.

Then, CPU 27 checks the lying-to-sleep state (step S6). Lying-to-sleep state determination unit 273 makes determination as to the lying-to-sleep state based on the result of sensing of the amount of body motion from body motion sensing unit 274 and provides a result of determination to ready-to-sleep state determination unit 276.

Then, CPU 27 makes determination as to the lying-to-sleep state (step S8). Ready-to-sleep state determination unit 276 determines whether or not it has received a notification about a result of determination as to the lying-to-sleep state from lying-to-sleep state determination unit 273.

Then, when CPU 27 makes determination as the lying-to-sleep state (YES in step S8), it makes determination as the ready-to-sleep state (step S10). Ready-to-sleep state determination unit 276 gives a notification that the result of determination indicates the ready-to-sleep state to sleep induction function controller 278. When the result of determination from presence/absence determination unit 271 indicates detection of presence of the user and lying-to-sleep state determination unit 273 makes determination as the lying-to-sleep state, ready-to-sleep state determination unit 276 makes determination as the ready-to-sleep state.

Though an example in which, when the result of determination from presence/absence determination unit 271 indicates detection of presence of the user and lying-to-sleep state determination unit 273 makes determination as the lying-to-sleep state, ready-to-sleep state determination unit 276 makes determination as the ready-to-sleep state is described in the present example, determination as the ready-to-sleep state may be made when the result of determination from presence/absence determination unit 271 indicates detection of presence of the user or determination as the ready-to-sleep state may be made when lying-to-sleep state determination unit 273 makes determination as the lying-to-sleep state.

Then, CPU 27 performs processing (step S12). Sleep induction function controller 278 performs prescribed processing as the function for induction to sleep.

Then, the process ends (return).

When CPU 27 determines in step S2 not as presence (NO in step S2), the process returns to step S0 and the processing above is repeated.

When CPU 27 makes determination in step S8 not as the lying-to-sleep state (NO in step S8), the process returns to step S0 and the processing above is repeated.

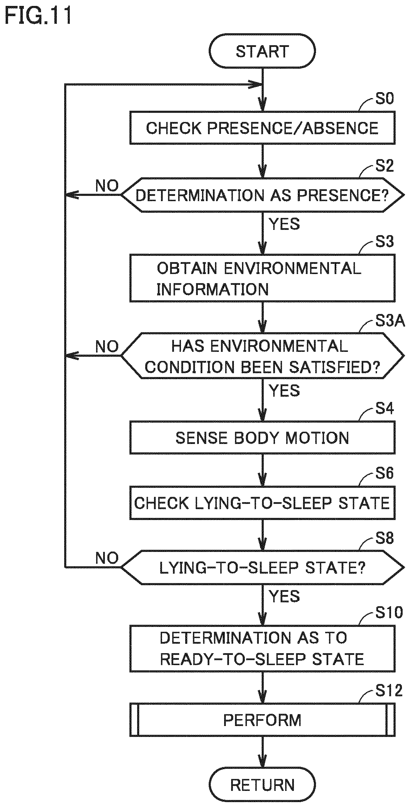

Fig. 11 is a diagram illustrating a flow of other sleep induction function start processing based on the embodiment.

As shown in Fig. 11, a difference from the processing in Fig. 10 resides in addition of step S3 and step S3A.

Specifically, when CPU 27 makes determination as presence in step S2 (YES in step S2), it obtains environmental information (step S3). Specifically, illuminance sensor 26 detects an illuminance value of a room as ambient environmental information. Illuminance sensor 26 provides the illuminance value of the room to ready-to-sleep state determination unit 276.

CPU 27 determines whether or not an environmental condition has been satisfied (step S3A). Ready-to-sleep state determination unit 276 determines whether or not the illuminance value provided from illuminance sensor 26 is equal to or smaller than a prescribed illuminance value.

When CPU 27 determines in step S3A that the environmental condition has been satisfied (YES in step S3A), it senses body motion (step S4). Body motion sensing unit 274 senses body motion of the user based on a signal from Doppler sensor 30. Body motion sensing unit 274 provides a result of sensing to lying-to-sleep state determination unit 273.

Then, CPU 27 checks the lying-to-sleep state (step S6). Lying-to-sleep state determination unit 273 makes determination as to the lying-to-sleep state based on the result of sensing of the amount of body motion from body motion sensing unit 274 and provides a result of determination to ready-to-sleep state determination unit 276.

Then, CPU 27 makes determination as to the lying-to-sleep state (step S8). Ready-to-sleep state determination unit 276 determines whether or not it has received a notification about a result of determination as the lying-to-sleep state from lying-to-sleep state determination unit 273.

Then, when CPU 27 makes determination as the lying-to-sleep state (YES in step S8), it makes determination as the ready-to-sleep state (step S10). Ready-to-sleep state determination unit 276 gives a notification that the result of determination indicates the ready-to-sleep state to sleep induction function controller 278.

Then, CPU 27 performs processing (step S12). Sleep induction function controller 278 performs prescribed processing as the function for induction to sleep.

Then, the process ends (return).

When CPU 27 determines in step S2 not as presence (NO in step S2), the process returns to step S0 and the processing above is repeated.

When CPU 27 makes determination in step S8 not as the lying-to-sleep state (NO in step S8), the process returns to step S0 and the processing above is repeated.

When CPU 27 determines in step S3A that the environmental condition has not been satisfied (NO in step S3A), the process returns to step S0 and the processing above is repeated.

### <Processing to Perform>

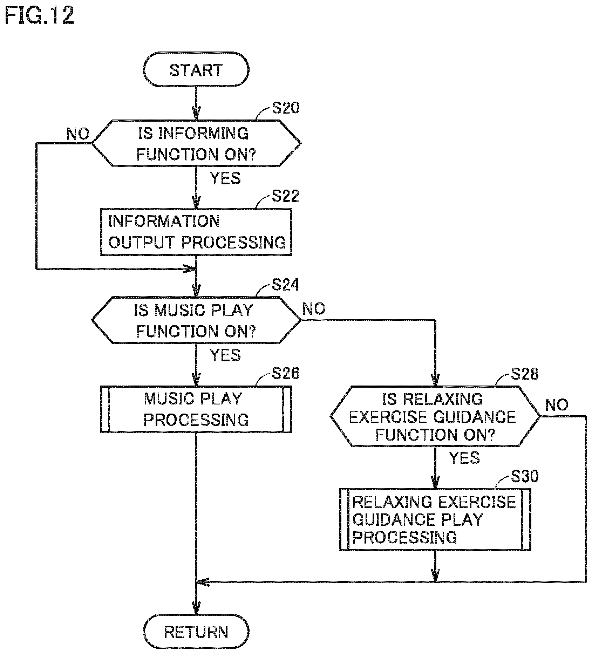

Fig. 12 is a diagram illustrating a flow of processing for performing the sleep induction function based on the embodiment.

As shown in Fig. 12, CPU 27 determines whether or not the informing function is ON (step S20).

When CPU 27 determines that the informing function is ON (YES in step S20), it performs processing to provide information (step S22).

Specifically, sleep induction function controller 278 gives through speaker 22, a notification about get-up set time of the alarm function included in the setting information based on the information stored in memory 23.

For example, information "get-up set time tomorrow is 7:00" is given. On the following day (next day) or when time passes the midnight, information on weather and a temperature in the following morning (next morning) may be given. For example, sleep induction function controller 278 instructs information obtaining unit 284 to obtain information on weather or a temperature by communicating with an external apparatus connected to the network through communication apparatus 24, so that the information is given through speaker 22. Alternatively, information obtaining unit 284 may obtain information in advance and have the information stored in memory 23, so that the information may be used. Though an example in which information on weather and a temperature is provided as information is described by way of example, limitation to the information on the weather and the temperature is not intended, but any information convenient for the user such as latest news, traffic information, or a memorandum may be applicable.

The informing function may provide, for example, information on the following day or the following morning while playing music. As output of the information on the following day or the following morning ends, play of the music may be stopped, so that the user can be induced to sleep while a relaxing effect of the user is enhanced.

Sleep induction function controller 278 can edit get-up set time in accordance with an instruction from the user, as a result of information output processing by using the informing function. Specifically, voice recognition unit 282 identifies audio input from the user through microphone 28 and provides the audio input to sleep induction function controller 278. For example, when the user says "change alarm to eight o'clock" and sleep induction function controller 278 determines that the audio input from the user indicates change of get-up set time, it changes get-up set time to eight o'clock

Then, CPU 27 determines whether or not the music play function is ON (step S24). Specifically, sleep induction function controller 278 determines whether or not information that the music play function included in the setting information is ON is included.

Then, when CPU 27 determines that the music play function is ON (YES in step S24), it performs music play processing (step S26). Details of the music play processing will be described later.

Then, the process ends (return).

When the music play function is determined as not being ON in step S24 (NO in step S24), whether or not the relaxing exercise guidance function is ON is determined (step S28). Sleep induction function controller 278 determines whether or not information that the relaxing exercise guidance function included in the setting information is on is included.

When CPU 27 determines that the relaxing exercise guidance play function is ON (YES in step S28), it performs relaxing exercise guidance play processing (step S30). Details of the relaxing exercise guidance play processing will be described later.

Then, the process ends (return).

When CPU 27 determines in step S28 that the relaxing exercise guidance function is not ON (NO in step S28), the process ends (return).

Though an example in which any one of the music play function and the relaxing exercise guidance function is performed is described in the present example, the music play processing and the relaxing exercise function guidance play processing may be performed in parallel by setting the music play function to ON and setting also the relaxing exercise guidance function to ON.

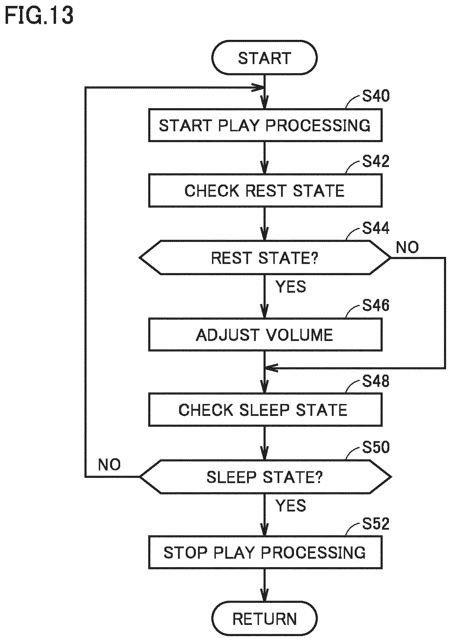

Fig. 13 is a diagram illustrating a flow of the music play processing based on the embodiment.

As shown in Fig. 13, CPU 27 starts play processing (step S40). Sleep induction function controller 278 plays a music file stored in memory 23 and provides output thereof from speaker 22.

Then, CPU 27 checks the rest state (step S42). Rest state determination unit 277 makes determination as to the rest state based on the amount of body motion calculated in body motion sensing unit 274.

Then, CPU 27 makes determination as to the rest state (step S44). Sleep induction function controller 278 determines whether or not the result of determination by rest state determination unit 277 indicates the rest state.

Then, when CPU 27 makes determination as the rest state (YES in step S44), it performs volume adjustment processing (step S46). When sleep induction function controller 278 determines that the result of determination by rest state determination unit 277 indicates the rest state, it performs processing for lowering the volume in music play provided through speaker 22.

Though an example in which sleep induction function controller 278 performs processing for lowering the volume is described in the present example, limitation thereto is not particularly intended. For example, a function of a cut filter to cut off a high-frequency component to reduce the high-frequency component may be performed. Processing for lowering the volume and the cut filter for cutting off the high-frequency component may be combined with each other. Alternatively, processing for adjusting a pitch may be performed. Alternatively, a type of the cut filter for cutting off the high-frequency component may be adjusted in accordance with a sound source. For example, cut filters for cutting off a high-frequency component may be varied in type between a sound source including vocal and a sound source without vocal.

Sleep induction function controller 278 may change the volume in music play provided in real time through speaker 22 or strength of the cut filter for cutting off the high-frequency component, in accordance with a parameter representing a duration of the rest state or a degree of the rest state in the result of determination by rest state determination unit 277.

Transition of the user to the sleep state can be promoted in accordance with the state of the user by performing the volume adjustment processing.

Then, CPU 27 checks the sleep state (step S48). Sleep state determination unit 272 determines whether or not the user is in the sleep state based on a detection signal from Doppler sensor 30.

Then, CPU 27 makes determination as to the sleep state (step S50). Sleep induction function controller 278 determines whether or not a result of determination provided from sleep state determination unit 272 indicates the sleep state. For example, when the sleep state of the user falls under a state other than the wake state, the deep sleep state, the light sleep state, or the REM sleep state, determination as the sleep state is made.

When CPU 27 makes determination as the sleep state in step S50 (YES in step S50), it stops play processing (step S52). Sleep induction function controller 278 stops processing for playing a music file stored in memory 23.

When determination as the sleep state is made, sleep induction function controller 278 may stop the play processing stepwise, rather than immediately stopping the play processing. For example, the volume may be varied stepwise in accordance with probability of determination as the sleep state, or when body motion is sensed or determination as the wake state is made while the volume is low, the volume may return to the original volume. Through the processing, the processing for playing a music file can also be prevented from being stopped by erroneous determination.

Then, the process ends (return).

When CPU 27 makes determination not as the sleep state in step S50 (NO in step S50), the process returns to step S40.

When CPU 27 makes determination not as the rest state in step S44 (NO in step S44), step S46 is skipped and the process proceeds to step S48.

When the sleep state of the user has been confirmed, the sleep state of the user can be stabilized by turning off the sleep induction function.

The relaxing exercise guidance play processing is also basically similar to the music play processing.

Specifically, CPU 27 starts play processing. Sleep induction function controller 278 plays a relaxing exercise guidance file stored in memory 23 and provides output thereof from speaker 22 and display 21.

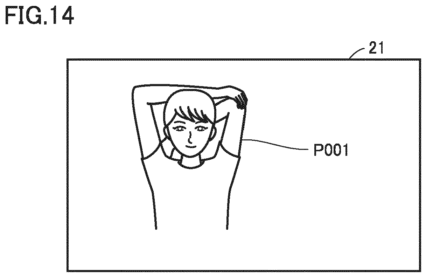

Fig. 14 is a diagram illustrating overview of the relaxing exercise guidance play processing based on the embodiment.

As shown in Fig. 14, display 21 shows an exercise guidance image P001 for relaxing the body. The user can do stretching exercise for relaxing the body in accordance with exercise guidance image P001. Together with representation of an exercise guidance image, music for relaxing the user may be played.

Though an example for inviting the user to do stretching exercise is described in the present example, any exercise is applicable so long as it is done for relaxing. For example, a guidance image can be shown to invite the user to perform an operation to take a deep breath by a breathing method and music can also be played.

Though an example in which sleep induction function controller 278 has a relaxing exercise guidance file stored in memory 23 played to provide output thereof from speaker 22 and display 21 of sleep alarm apparatus 2 is described in the present example, another device may be used without being particularly limited as such. Specifically, sleep induction function controller 278 may transmit data to terminal 8 through communication apparatus 24 and may have a relaxing exercise guidance file played to provide output thereof from display 82 and a speaker (not shown) of terminal 8. This is also similarly applicable to the music play function.

As described with reference to the flowchart in Fig. 13, CPU 27 makes determination as to the rest state, and when it makes determination as the rest state, it performs the volume adjustment processing. When sleep induction function controller 278 determines that the result of determination by rest state determination unit 277 indicates the rest state, it performs processing for lowering the volume in music play provided through speaker 22. Though an example in which sleep induction function controller 278 performs processing for lowering the volume is described in the present example, limitation thereto is not particularly intended. For example, a function of a cut filter to cut off a high-frequency component to reduce the high-frequency component may be performed. Processing for lowering the volume and the cut filter for cutting off the high-frequency component may be combined with each other. Alternatively, processing for adjusting a pitch may be performed. Alternatively, a type of the cut filter for cutting off the high-frequency component may be adjusted in accordance with a sound source. For example, cut filters for cutting off a high-frequency component may be varied in type between a sound source including vocal and a sound source without vocal.

Sleep induction function controller 278 may change the volume in music play provided in real time through speaker 22 or strength of the cut filter for cutting off the high-frequency component, in accordance with a parameter representing a duration of the rest state or a degree of the rest state indicated in the result of determination by rest state determination unit 277.

Sleep induction function controller 278 may change the guidance image or audio information or may interactively support how to breath, in response to a non-moving state in stretching exercise in accordance with a parameter representing a duration of the rest state or a degree of the rest state indicated in the result of determination by rest state determination unit 277 or sensing of a breathing state of the user.

Transition of the user to the sleep state can be promoted in accordance with the state of the user, by performing the volume adjustment processing.

Then, CPU 27 makes determination as to the sleep state, and when it makes determination as the sleep state, it stops play processing. Sleep induction function controller 278 stops processing for playing a relaxing exercise guidance file stored in memory 23.

When the sleep state of the user has been confirmed, the sleep state of the user can be stabilized by turning off the sleep induction function.

An application executable by a personal computer may be provided as a program in the present embodiment. The program according to the present embodiment may be incorporated as some functions of various applications executed on the personal computer.

It should be understood that the embodiment disclosed herein is illustrative and non-restrictive in every respect. The scope of the present invention is defined by the terms of the claims rather than the description above and is intended to include any modifications within the scope and meaning equivalent to the terms of the claims.

### REFERENCE SIGNS LIST

1 sleep management system; 2 sleep alarm apparatus; 4 network; 6 server; 8 terminal; 20 clock; 21, 82 display; 22 speaker; 23, 62, 86 memory; 24, 64, 84 communication apparatus; 26 illuminance sensor; 28 microphone; 29, 88 input apparatus; 30 Doppler sensor; 32, 66, 89 internal bus; 200 alarm setting screen; 271 absence determination unit; 272 sleep state determination unit; 273 lying-to-sleep state determination unit; 274 body motion sensing unit; 275 representation controller; 276 ready-to-sleep state determination unit; 277 rest state determination unit; 278 sleep induction function controller; 280 sleep induction function execution determination unit; 282 voice recognition unit; 284 information obtaining unit; 286 notification controller

## Claims

1. An information processing apparatus comprising:
a sensing unit that senses body motion of a user present within a prescribed area;
a determination unit that determines whether the user is in a ready-to-sleep state based on a result of sensing by the sensing unit; and
a processing performing unit that performs first processing when a result of determination by the determination unit indicates that the user has entered the ready-to-sleep state.

2. The information processing apparatus according to claim 1, wherein
the determination unit determines that the user has entered the ready-to-sleep state based on at least one of sensing of the user within the prescribed area and the user being in a lying-to-sleep state based on the result of sensing by the sensing unit.

3. The information processing apparatus according to claim 2, further comprising an ambient environment obtaining unit that obtains a state of an environment around the information processing apparatus, wherein
when the result of determination by the determination unit indicates that the user has entered the ready-to-sleep state and when the state of the environment obtained by the ambient environment obtaining unit satisfies an ambient environment condition, the processing performing unit performs the first processing.

4. The information processing apparatus according to claim 3, wherein
the ambient environment obtaining unit obtains as the state of the environment, information on ambient brightness.

5. The information processing apparatus according to claim 4, wherein
the information on ambient brightness includes an illuminance value.

6. The information processing apparatus according to any one of claims 2 to 5, further comprising a communication unit provided to communicate with an external apparatus, wherein
the processing performing unit provides audio output of information on a next day or a next morning obtained from the external apparatus through the communication unit, as the first processing.

7. The information processing apparatus according to claim 6, further comprising a notification unit that performs a notification operation when time set in advance comes, wherein
the information on the next day or the next morning includes time at which the notification operation is performed.

8. The information processing apparatus according to claim 7, further comprising a voice recognition unit that accepts audio input and carries out voice recognition, wherein
the processing performing unit changes time at which the notification operation is performed, in accordance with a result of the voice recognition by the voice recognition unit.

9. The information processing apparatus according to any one of claims 6 to 8, wherein
the information on the next day or the next morning includes information on weather on the next day or in the next morning.

10. The information processing apparatus according to any one of claims 1 to 9, wherein
the sensing unit includes a Doppler sensor, and
the information processing apparatus further comprises a body motion sensing unit that senses body motion of the user based on an output from the Doppler sensor.

11. The information processing apparatus according to claim 10, further comprising a sleep state measurement unit that measures a sleep state of the user following the first processing, based on the output from the Doppler sensor.

12. The information processing apparatus according to claim 10, wherein when the user is determined as having entered a rest state based on measurement of the rest state of the user, the processing performing unit performs second processing for adjusting contents of the first processing.

13. The information processing apparatus according to claim 12, wherein
the processing performing unit performs third processing for inducing the user to a sleep state when the user is not in the sleep state, and
the processing performing unit stops the third processing when the user enters the sleep state.

14. The information processing apparatus according to claim 13, wherein the third processing includes music play processing.

15. The information processing apparatus according to claim 13 or 14, wherein
the third processing includes voice guidance processing for indicating a breathing method or prescribed exercise.

16. The information processing apparatus according to claim 1, wherein the sensing unit senses body motion of the user present within the prescribed area over a whole period of twenty-four hours.

17. An information processing method comprising:
sensing body motion of a user present within a prescribed area;
determining whether the user is in a ready-to-sleep state based on a result of sensing; and
performing first processing when the user is determined as having entered the ready-to-sleep state.

18. An information processing program executed by a computer including a sensor that senses a signal in accordance with motion of a user, the information processing program causing the computer to perform:
sensing body motion of the user present within a prescribed area based on the signal from the sensor;
determining whether the user is in a ready-to-sleep state based on a result of sensing; and
performing first processing when the user is determined as having entered the ready-to-sleep state.

19. An information processing system comprising:
a sensing unit that senses body motion of a user present within a prescribed area;
a determination unit that determines whether the user is in a ready-to-sleep state based on a result of sensing by the sensing unit; and
a processing performing unit that performs first processing when a result of determination by the determination unit indicates that the user has entered the ready-to-sleep state.
